# EUROPÄISCHE PATENTANMELDUNG

(11) **EP 0 901 791 A1**
(43) Veröffentlichungstag der Anmeldung: **17.03.1999**
(21) Anmeldenummer: 98117366.9
(22) Anmeldetag: 14.09.1998
(51) Int. Cl.: A61K 35/78

(54) **Verwendung eines Weissdornfrüchte und/oder -blätter/Blütenextraktes**

(30) Priorität: 15.09.1997 DE 19740509
(71) Anmelder: ROBUGEN GMBH PHARMAZEUTISCHE FABRIK, 73730 Esslingen-Zell (DE)
(72) Erfinder: Belz, Gustav, Prof. Dr.med., 65189 Wiesbaden (DE); Berndt, Dieter, 73733 Esslingen (DE); Hempel, Bernd, Dr., 73730 Esslingen (DE)
(74) Vertreter: Kinzebach, Werner, Dr.

(57) **Zusammenfassung**

Die vorliegende Erfindung betrifft die Verwendung eines Weißdornfrüchte- und/oder -blätter/blütenextraktes zur Steigerung der Leistungsfähigkeit des Herzens und gleichzeitigen Erhöhung des Blutdrucks, insbesondere zur Behandlung von Herzbeschwerden bei Hypotonie. Die vorliegende Erfindung betrifft auch die Verwendung eines Weißdornfrüchte- und/oder -blätter/blütenextraktes zur Förderung der Diurese.

## Beschreibung

Die vorliegende Erfindung betrifft die Verwendung eines Weißdornfrüchte- und/oder -blätter/blütenextraktes zur Steigerung der Leistungsfähigkeit des Herzens und gleichzeitigen Erhöhung des Blutdrucks und zur Förderung der Diurese.

Wertvolle Inhaltsstoffe machen den Weißdorn (*Crataegus* L.) zu einer beliebten drogenliefernden Pflanzengattung. In der Regel bedient man sich der Weißdornblüten (Crataegi flos), der Weißdornblätter mit Blüten (Crataegi folium cum flore) oder der Weißdornfrüchte (Crataegi fructus). Als drogenliefernde Arten werden häufig die Welsche oder Italienische Mispel (*Crataegus azarolus* L.), der Zweigriffelige Weißdorn, der auch als Hagedorn oder Mehldorn bezeichnet wird (*Crataegus laevigata* (POIRET) DC oder *Crataegus oxyacantha* L.), der Japanische Weißdorn (*Crataegus cuneata* SIEB. et ZUCC.), der Eingriffelige Weißdorn (*Crataegus monogyna* JACQUIN emend. LINDMAN), der Schwarzdorn (*Crataegus nigra* WALDST. et KIT.), der Fünfgriffelige Weißdron (*Crataegus pentagyna* WALDST. et KIT. EX WILLD.) und der Chinesische Weißdorn (*Crataegus pinnatifida* BUNGE) genannt. Die Ausdrücke "Weißdorn" und "Crataegus" werden im folgenden synonym vewendet.

Bei den Inhaltsstoffen des Weißdorns handelt es sich in erster Linie um Flavonoide und Flavanverbindungen, die in den Blättern, Blüten und Früchten aller bisher untersuchten Weißdornarten gefunden wurden. Neben geringen Anteilen frei vorliegender Flavonole und Flavone bestimmen Flavonglykoside und Flavonolglykoside das Flavonoidmuster. Sie leiten sich in der Regel vom Apigenin und Luteolin bzw. vom Quercetin, Kämpferol und 8-Methoxykämpferol ab. Neben monomeren Flavanen, wie (+)-Catechin und (-)-Epicatechin, finden sich dimere und oligomere Dehydrocatechine, die bei Behandlung mit Mineralsäuren Cyanidine bilden und daher Procyanidine genannt werden. Oligomere Procyanidine umfassen definitionsgemäß bis zu 10 Monomereinheiten, während Ketten mit einem Polymerisationsgrad von mehr als 10 als polymere Procyanidine bezeichnet werden. Je nach Verknüpfung und der daraus resultierenden Stereochemie unterscheidet man verschiedene Procyanidin-Typen, von denen der sog. B-2-Typus, d.h. 4,8-all-2,3-cis-Procyanidin, häufig auftritt (siehe Hagers Handbuch der pharmazeutischen Praxis, Band 4, 5. Auflage, Springer-Verlag Berlin Heidelberg 1992, S. 1041 - 1043).

Wohl aufgrund dieser Inhaltsstoffe haben drogenliefernde Arten des Weißdorns breite Anwendung in der volkstümlichen Medizin gefunden. Insbesondere die getrockneten, reifen Früchte, gegebenenfalls zu Kompott oder Marmelade verarbeitet oder als Teeaufguß zubereitet, sollen bei leichten Schleimhautentzündungen im Mund- und Rachenraum, bei Durchfallerkrankungen und Verdauungsbeschwerden, Unterleibsschmerzen, stechenden Schmerzen in der Herzgegend, Hyperlipidämie, Bandwurmerkankungen oder Bluthochdruck zur Anwendung kommen.

Aufgrund der guten Verträglichkeit von Crataeguszubereitungen hat auch die moderne Medizin diese Droge entdeckt und einige der schon traditionell genutzten, wertvollen pharmakologischen Eigenschaften durch klinische Studien belegt.

So werden in einer am 5. Mai 1998 korrigiert im Bundesanzeiger veröffentlichten Monographie des Bundesgesundheitsamtes folgende Anwendungsgebiete für die Blätter mit Blüten und/oder Früchte des Weißdorns sowie deren Zubereitungen in wirksamer Dosierung angegeben: Nachlassende Leistungsfähigkeit des Herzens entsprechend den Stadien I - II nach NYHA (New York Heart Association), Druck- und Beklemmungsgefühl in der Herzgegend, noch nicht digitalisbedürftiges Altersherz und leichte Formen von bradykarden Herzrhytmusstörungen.

In einer weiteren, am 19. Juli 1994 im Bundesanzeiger veröffentlichten Monographie des Bundesgesundheitsamtes werden für Zubereitungen aus Weißdornblättern mit Blüten folgende pharmakodynamische Wirkungen angegeben: Positiv inotrope, positiv dromotrope und negativ bathmotrope Wirkung, Zunahme der Koronar- und Myokarddurchblutung sowie Senkung des peripheren Gefäßwiderstandes. Auch hier wird als klinisches Anwendungsgebiet eine nachlassende Leistungsfähigkeit des Herzens entsprechend Stadium II nach NYHA genannt. Bezüglich der Weißdornfrüchte wird in der gleichen Monographie festgestellt, daß ähnliche pharmakodynamische Wirkungen angenommen werden können, wie sie für Weißdornblätter mit Blüten nachgewiesen wurden. Dementsprechend sollen Weißdornfrüchte bzw. Zubereitungen aus Weißdornfrüchten zur Förderung der Durchblutung der Herzkranzgefäße, bei Herzerweiterung und Herzschwäche, bei Herz-Kreislauf-Störungen, gegen hohen Blutdruck und gegen Arteriosklerose angewendet werden.

Darüber hinaus offenbart die WO 95/03816 einen aus Blättern mit Blüten geeigneter Crataegusarten gewonnenen Extrakt bzw. bestimmte Fraktionen davon und deren Anwendung zur Verhinderung des plötzlichen Herztodes und reperfusionsbedingter kardiovaskulärer Schädigungen und anderer reperfusionsbedingter, lebensbedrohlicher pathologischer Zustände.

Schließlich beschreibt die WO 96/14078 einen speziellen Extrakt aus Blättern, Blüten und/oder Früchten von Crataegus monogyna oder Crataegus oxyacantha, dessen Herstellungsverfahren ein Erhitzen der wässrigen, durch herkömmliche Extraktion gewonnenen Lösung zur Bildung höhermolekularer kondensierbarer Flavane und ein Abfiltrieren der beim Erhitzen ausgefällten Bestandteile umfaßt. Der so hergestellte Extrakt ist insbesondere zur Behandlung allergischer Erkrankungen geeignet.

Der Stand der Technik zeigt somit, daß Crataegusextrakte bisher zur Behandlung verschiedenster Erkrankungen verwendet worden sind. Über die Art des Extraktes, Dosis und Indikation werden dabei häufig sich widersprechende Angaben gemacht; jedenfalls herrscht Unklarheit.

Der Erfindung liegt daher die Aufgabe zugrunde, die Therapie mit Crataegus aufzuklären, von Widersprüchen zu befreien und zu verbessern.

Bei Dosis-Wirkungsstudien mit verschiedensten Crataeguspräparaten wurde überraschenderweise gefunden, daß Weißdornfrüchte- und/oder -blätter/blütenextrakte ab einer bestimmten Dosis eine Erhöhung des Blutdrucks und eine Förderung der Diurese bewirken.

Ein Gegenstand der vorliegenden Erfindung ist daher die Verwendung eines Weißdornfrüchte- und/oder -blätter/blütenextraktes zur Steigerung der Leistungsfähigkeit des Herzens und gleichzeitigen Erhöhung des Blutdrucks.

Ein weiterer Gegenstand der vorliegenden Erfindung ist die Verwendung eines Weißdornfrüchte- und/oder -blätter/blütenextraktes zur Förderung der Diurese.

Diese Indikationen sind neu und angesichts des Standes der Technik überraschend, denn der Stand der Technik lehrt bezüglich der Beeeinflussung des Blutdruckes durch Crataegus das Gegenteil, nämlich die blutdrucksenkende Wirkung (vergleiche beispielsweise in die eingangs diskutierten Monographien).

Zur Beurteilung der Leistungsfähigkeit des Herzens sind dem Fachmann eine Reihe von Parametern bekannt, die er bestimmen und auswerten kann. So kann sich eine Steigerung der Leistungsfähigkeit des Herzens beispielsweise in einer Erhöhung der Arbeitstoleranz, einer Erniedrigung des Druckfrequenzproduktes, einer Erhöhung der Ejektionsfraktion, d.h. der Blutmenge, welche die linke Herzkammer in der Systole auswirft, einer Erhöhung der anaeroben Schwelle, d.h. der Toleranz des Myokards gegenüber Sauerstoffmangel, einer verbesserten Kontraktilität des Myokards, einer erhöhten Koronar- und Myokarddurchblutung sowie einer gesteigerten Reiz- und Erregungsleitungsfähigkeit des Herzens äußern. Herzschlag- und -zeitvolumen sowie die Herzfrequenz stellen weitere geeignete Parameter dar. Die vorstehend geschilderten Effekte können bekannterweise *in vitro*, beispielsweise an Langendorff-Herzpräparaten aus Meerschweinchen oder Kaninchen, oder an verschiedenen Hypoxie-Modellen untersucht werden. Auch *in vivo* können entsprechende, ebenfalls bekannte Messungen vorgenommen und eine Leistungssteigerung des Herzens festgestellt werden.

Auch zur Beurteilung des Blutdrucks sind dem Fachmann eine Reihe von Parametern bekannt, die er bestimmen und auswerten kann. Hierzu zählen beispielsweise der systolische und diastolische Blutdruck, der arterielle Mitteldruck und der aus diesen Parametern ableitbare totale periphere Widerstand. Unter einer Erhöhung des Blutdrucks im erfindungsgemäßen Sinn wird die Erhöhung eines oder mehrerer dieser Parameter verstanden.

Beide Wirkungen, die Steigerung der Leistungsfähigkeit des Herzens und die Erhöhung des Blutdrucks, treten bei der erfindungsgemäßen Verwendung des Weißdornextraktes im wesentlichen gleichzeitig auf, d.h. beide Wirkungen sind zumindest zeitweise gleichzeitig zu beobachten. Dies soll nicht ausschließen, daß der jeweilige Wirkungseintritt zu unterschiedlichen Zeitpunkten erfolgen kann. Auch die zeitlichen Verläufe beider Wirkungen können sich voneinander unterscheiden, so daß die jeweilige maximale Wirkung zu unterschiedlichen Zeitpunkten erreicht werden kann.

Die erfindungsgemäße Verwendung des Weißdornextraktes kann auch bei normaler Herzleistungsfähigkeit von Vorteil sein. In der Regel betrifft sie allerdings ein Herz, dessen Leistungsfähigkeit im Vergleich zum Normalzustand verringert ist. Eine bevorzugte erfindungsgemäße Verwendung des Weißdornextraktes ist daher die Behandlung von Herzbeschwerden bei Hypotonie. Im Einklang mit der oben beschriebenen Verwendung zur Steigerung der Leistungsfähigkeit des Herzens ist erfindungsgemäß die Verwendung zur Behandlung solcher Herzbeschwerden von Vorteil, die mit einer Herzleistungsschwäche, vorzugsweise leichter Herzinsuffizienz (Stadium I und II nach NYHA), einem noch nicht digitalisbedürftigem Altersherz, Druck- und Beklemmungsgefühlen in der Herzgegend und/oder leichten Formen von Herzrhytmusstörungen, zusammenhängen.

Die erfindungsgemäße Verwendung ist besonders dann von Vorteil, wenn Herzbeschwerden bei Patienten auftreten, die zumindest eine Neigung zur hypotonen Dysregulation aufweisen und bei denen eine kreislaufstabilisierende Wirkung erwünscht ist. Hierzu zählen beispielsweise Patienten, bei denen bereits vor der Anwendung ein zu niedriger Blutdruck festgestellt worden ist oder während der Anwendung erwartet wird. Insbesondere dann, wenn eine langsam einsetzende und lang andauernde blutdruckerhöhende Wirkung erwünscht ist, kommt die erfindungsgemäße Verwendung vorteilhaft zum Zuge. Erfindungsgemäß gelingt es daher, durch Verabreichung des Weißdornextraktes sowohl herz- als auch hypotoniebedingten Beschwerden entgegenzuwirken.

Auch zur Beurteilung der Diurese, d.h. der Harnausscheidung, sind dem Fachmann eine Reihe von Parametern geläufig, die er bestimmen und auswerten kann. Hierzu zählt beispielsweise die Messung der thorakalen Grundimpedanz. Die Impedanz ist als Meßgröße von der Leitfähigkeit des Organismus abhängig, welche wiederum von der Menge intrakorporaler Elektrolyte sowie von dem damit in Wechselwirkung stehenden Flüssigkeitshaushalt bestimmt werden. So führen höhere intrakorporale Elektrolytdichten bzw. ein geringerer Flüssigkeitsgehalt als Folge einer verstärkten Diurese zu einem Anstieg der thorakalen Grundimpedanz.

Aufgrund der diuresefördernden Wirkung betrifft die erfindungsgemäße Verwendung des Weißdornextraktes auch die Behandlung von Erkrankungszuständen, die ursächlich mit einer verminderten Diurese zusammenhängen oder auf die sich eine verstärkte Diurese positiv auswirken kann. Beispiele für derartige Erkrankungszustände sind Ödeme sowie hydropische Zustände unterschiedlicher Genese.

Es versteht sich, daß auch die auf die Förderung der Diurese bezogene erfindungsgemäße Verwendung des Weißdornextraktes bei Patienten mit hypotonen Dysregulationen vorteilhaft zum Zuge kommt. Eine derartige Indikation ist von Vorteil, da herkömmliche Diuretika in der Regel zu einer Senkung des Blutdrucks führen und daher bei bestehender oder zu erwartender Hypotonie nicht oder nur mit großen Risiken für den Patienten anwendbar sind.

Zur Herstellung des erfindungsgemäßen Weißdornfrüchte- und/oder -blätter/blütenextraktes sind alle Crataegusarten geeignet. Insbesondere greift man auf die oben genannten und vorzugsweise die im deutschen Arzneibuch aufgeführten Crataegusarten zurück. Ganz besonders bevorzugt sind Extrakte aus Crataegus oxyacantha und Crataegus monogyna.

In der Regel verwendet man die Früchte, die Blätter mit Blüten, d.h. die blühenden Zweigspitzen, und/oder die Blüten der Weißdornpflanze. Erfindungsgemäß bevorzugt sind die Weißdornfrüchte, d.h. die Weißdornbeeren, insbesondere zur Steigerung der Leistungsfähigkeit des Herzens und gleichzeitigen Erhöhung des Blutdrucks und zur Behandlung entsprechender Indikationen. Es können Beeren unterschiedlicher Reifestadien verwendet werden. Maximale Ausbeuten an Extrakt erzielt man in der Regel mit vollreifen Früchten, welche die größere Masse aufweisen. Es kann allerdings auch von Vorteil sein, Beeren eines früheren Reifestadiums zu wählen, weil der Gehalt an Procyanidinen vom Reifestadium abhängig und im unreifen Stadium relativ hoch ist.

Zur Herstellung des Extraktes sind dem Fachmann eine Vielzahl von Verfahren bekannt. Üblicherweise geht man von getrockneten Pflanzenteilen aus, d.h. die frisch geernteten, vollreifen Früchte, die blühenden Zweigspitzen und/oder die abgetrennten Blüten werden zunächst einem gängigen Trocknungsverfahren, beispielsweise einer Luft- oder Wärmetrocknung, vorzugsweise einer Gefriertrocknung, unterzogen. Werden die Weißdornfrüchte verwendet, sind erfindungsgemäß Früchte ohne vorherige Trocknung im frischen oder triefgefrorenen Zustand bevorzugt. Es kann auch von Vorteil sein, eine Keimreduzierung und/oder Entwesung vorzunehmen.

Aus dem so gewonnenen Feststoffgemisch, der Droge, werden dann verschiedene Komponenten des Gemisches, vorzugsweise die für die angestrebte Wirkung verantwortlichen Inhaltsstoffe, herausgelöst, d.h. extrahiert. Das zur Extraktion eingesetzte Lösungsmittel oder Lösungsmittelgemisch, auch Menstruum genannt, richtet sich in erster Linie nach den Löslichkeitseigenschaften der zu extrahierenden Inhaltsstoffe. Sollen im wesentlichen hydrophile Inhaltsstoffe extrahiert werden, verwendet man vorzugsweise ein hydrophileres Lösungsmittel oder Lösungsmittelgemisch, während zur Extraktion von im wesentlichen hydrophober Inhaltsstoffe ein vergleichsweise hydrophoberes Lösungsmittel oder Lösungsmittelgemisch bevorzugt ist.

Geeignet sind flüssige Extraktionsmittel, beispielsweise Wasser, C₁₋₄-Alkohole, wie Methanol, Ethanol, n-Propanol oder iso-Propanol, Ketone, wie Aceton, Ester, wie Ethylacetat, Ether, wie Diethylether, oder halogenierte Kohlenwasserstoffe, wie Dichlormethan, sowie Gemische davon, und bei Raumtemperatur gasförmige Extraktionsmittel, wie CO₂, NH₃, Distickstoffoxid oder Edelgase. Erfindungsgemäß setzt man vorzugsweise wässrigalkoholische Gemische, aber auch reine Alkohole ein. Als Alkohole eignen sich beispielsweise Methanol und ganz besonders Ethanol. Bevorzugte Alkoholkonzentrationen liegen im Bereich von 30 - 100 Vol-%. Natürlich lässt sich durch die Wahl der Alkoholkonzentration die Zusammensetzung der extrahierten Inhaltsstoffe beeinflussen. 50-70 Vol-%iger Alkoholol extrahiert neben lipophilen Stoffen auch hohe Anteile an polaren Aminosäuren und Zuckern, höherprozentiger Alkohol dagegen auch ätherische Öle und Harze.

Vor dem Zusatz des Lösungsmittels oder des Lösungsmittelgemisches werden die getrockneten Pflanzenteile gegebenenfalls gereinigt, zerkleinert, gesichtet und/oder verschnitten. Insbesondere das Zerkleinern ist für die nachfolgende Extraktion von Bedeutung, da es die Oberfläche und somit die Angriffsfläche für die Extraktionsflüssigkeit vergrößert. Allerdings sollte die Droge auch nicht zu fein gemahlen sein, da dies das Abtrennen der Extraktionsflüssigkeit vom Drogenrückstand erschweren kann. Werden die Früchte verwendet, sind erfindungsgemäß die unzerkleinerten, frischen oder tiefgefrorenen Früchte bevorzugt, die erst während der Mazeration zerschlagen werden.

Dann wird die Droge mit dem Lösungsmittel oder Lösungsmittelgemisch zu einem Ansatzgemisch zusammengegeben und einem der bekannten Extraktionsverfahren unterzogen. Hierbei kann man zwei grundsätzlich verschieden ablaufende Verfahren unterscheiden. Zum einen handelt es sich um Verfahren, die zur Einstellung eines Konzentrationsgleichgewichtes zwischen Miscella, der extrahierten Flüssigkeit, und Drogenrückstand führen. Hierzu zählen beispielsweise verschiedene Mazerationsarten, die Digestion und die Wirbel- oder Ultraschallextraktion. Zum anderen handelt es sich um Verfahren, die bis zur vollständigen Erschöpfung der Droge führen können. Hierzu zählen beispielsweise die Perkolation, Reperkolation, Soxhletverfahren, Gegenstromextraktion, Evakolation und Diakolation sowie die Extraktion mit überkritischen Gasen, beispielsweise mit CO₂. Nach Beendigung dieser Verfahren werden Drogenrückstand und Miscella voneinander getrennt, wobei man den Rückstand noch auspressen kann, um weitere Miscella zu gewinnen.

Die so gewonnene Miscella kann dann weiteren herkömmlichen Reinigungsschritten unterzogen werden, um eventuell vorhandene Verunreinigungen zu entfernen.

Die im Extrakt vorhandenen Anteile an Drogeninhaltsstoffen sowie an Lösungsmittel hängen einerseits von der Ausgestaltung des oben beschriebenen Extraktionsverfahrens ab, können aber andererseits auch durch eine anschließende Behandlung der Miscella beeinflusst werden. So wird die Konzentration der Extraktivstoffe in der Miscella in der Regel durch Verdunsten oder Verdampfen von Lösungsmitteln auf eine gewünschte, höhere Konzentration eingestellt. Dies kann zur Herstellung zähflüssigerer Extrakte oder Konzentrate als Zwischenstufe zur Weiterverarbeitung zu diversen Arzneimittelformen, zur Herstellung von Trockenextrakten oder zur Konzentrierung auf einen erwünschten Trockensubstanzgehalt dienen. So lassen sich beispielsweise Trockenextrakte herstellen, indem das Menstruum vollständig verdampft und der verbleibende Rückstand anschließend getrocknet wird, so daß ein Feuchtigkeitsgehalt von vorzugsweise weniger als 5 % verbleibt. Fluidextrakte werden erhalten, indem man das oben beschriebene Verfahren so führt, daß letztlich ein Teil Droge höchstens zwei Teilen Fluidextrakt entspricht.

Erfindungsgemäß geeignete Extrakte sind Dünn- und Dickextrakte (Extractum tenue bzw. spissum) sowie Trocken- und Fluidextrakte (Extractum siccum bzw. fluidum). Ein zunächst gewonnener Trockenextrakt kann natürlich durch Zusatz eines Lösungsmittels oder Lösungsmittelgemisches wieder in einen gießfähigen Extrakt überführt werden.

Den Gehalt des Extraktes an Drogeninhaltsstoffen kann der Fachmann anhand bekannter analytischer Methoden bestimmen. Zur Analyse eines Weißdornextraktes kann man beispielsweise den Gehalt an Flavonoiden photometrisch bestimmen (DAB 10), wobei man z.B. die Extinktion von Hyperosid als Berechnungsgrundlage wählen kann; die durch eine Dünnschichtchromatographie aufgetrennten Inhaltsstoffe quantitativ, beispielsweise fluorimetrisch, auswerten; den Gehalt an oligomeren Procyanidinen photometrisch bestimmen, wobei man in der Regel die Extinktion von Cyanidin-Hydrochlorid als Berechnungsgrundlage wählt; oder die Flavone, Flavonole, Flavane sowie sämtliche Phenolcarbonsäuren umfassenden Gesamtphenole photometrisch, beispielsweise auf Basis der Extinktion von Catechin, Epicatechin oder auch Hyperosid, quantitativ auswerten. Üblicherweise werden deshalb Mengenangaben für Weißdornextrakte auf einen bestimmten Gehalt an Inhaltsstoffen bezogen, wobei der analytisch bestimmte Inhaltsstoff und die Berechnungsgrundlage zu bezeichnen sind.

Das gleiche gilt auch für Dosisangaben. Die erfindungsgemäßen Weißdornfrüchte- und/oder -blätter/blütenextrakte können eine Tagesdosis umfassen, die einer Verabreichung von etwa 15 bis 1200 mg Procyanidin-Äquivalenten (bestimmt als oligomere Procyanidine, berechnet als Cyanidin-Hydrochlorid) entspricht. Eine derartige Tagesdosis entspricht etwa 1,7 bis 140 mg Flavonoiden (bestimmt nach DAB 10, berechnet als Hyperosid) oder etwa 3 bis 280 mg Gesamtflavonoiden (bestimmt als Gesamtphenole, berechnet als Hyperosid). Bei den Weißdornfrüchten, d.h. den Beeren, bietet sich darüber hinaus eine andere Bezugsgröße an. Die oben angegebene Tagesdosis entspricht der Aufnahme von etwa 3 bis 200 g Frischbeeren. Erfindungsgemäß bevorzugt sind Tagesdosen im Bereich von 36 bis 600 mg Procyanidin-Äquivalenten, was etwa 6 bis 100 g Frischbeeren entspricht, und insbesondere von 60 bis 300 mg Procyanidin-Äquivalenten, was etwa 10 bis 50 g Frischbeeren entspricht. Die vorstehend genannten Dosisangaben beziehen sich auf ein Durchschnittsindividuum mit einem mittleren Körpergewicht von etwa 75 kg. Je nach tatsächlichem Körpergewicht kann die Tagesdosis entsprechend angepaßt werden.

Die zu verabreichende Tagesdosis hängt letztendlich vom Zustand des zu behandelnden Individuums ab. Dieser bestimmt wiederum die erwünschte Wirkung, so daß der Arzt unter Berücksichtigung weiterer, die Wirkung beeinflussender Parameter, beispielsweise des Verabreichungsweges, die Tagesdosis festlegen kann. Allerdings sollte die zur Verfügung gestellte Verabreichungsform die wesentlichen Eigenschaften der angestrebten Anwendung berücksichtigen. Erfindungsgemäß werden deshalb Arzneiformen bevorzugt, mit denen sich die oben angegebenen, bevorzugten Tagesdosen verabreichen lassen. In diesem Sinne sind recht konzentrierte Extrakte, d.h. Extrakte mit einem hohen Anteil an wirksamen Inhaltsstoffen, beispielsweise mit einem Droge-Extrakt-Verhältnis von 5:1, von Vorteil.

Gegenstand der vorliegenden Erfindung ist daher auch die Verwendung eines Weißdornfrüchte- und/oder -blätter/blütenextraktes zur Herstellung eines pharmazeutischen Mittels zu den oben genannten Zwecken. Erfindungsgemäß bevorzugt sind pharmazeutische Mittel zur oralen Verabreichung. So können die Weißdornextrakte gegebenenfalls in Kombination mit einem oder mehreren, pharmazeutisch verträglichen Träger- und/oder Hilfsstoffen als flüssige Arzneiform, beispielsweise Tropfen, oder feste Arzneiformen, beispielsweise Tabletten, Dragees und Hart- oder Weichgelatinekapseln, angeboten werden. Geeignete pharmazeutisch verträgliche Träger- und/oder Hilfsstoffe werden in der Regel auf die Arzneiform und den Verabreichungsweg, aber auch auf die Art des Wirkstoffs, d.h. den erfindungsgemäßen Weißdornextrakt abgestimmt. Es kann sich dabei um Verdünnungsmittel, Puffer, Geschmackskorrigienten, Tenside, Verdickungsmittel, Gleitmittel, Konservierungsmittel einschließlich Antioxidantien, Emulgatoren und dergleichen handeln.

Die Arzneiform kann neben dem Weißdornextrakt auch weitere, therapeutisch wertvolle Bestandteile umfassen, die mit dem Extrakt verträglich sind. Hierzu zählen beispielsweise weitere Kardiaka. Bevorzugt sind Kombinationen des Weißdornextraktes mit weiteren blutdrucksteigernden Mitteln insbesondere dann, wenn sich die jeweiligen blutdrucksteigernden Wirkungen ergänzen oder sogar potenzieren. Für die Kombination mit dem Weißdornextrakt erfindungsgemäß bevorzugt sind daher Wirkstoffe, deren blutdrucksteigernde Wirkung rasch einsetzt und nach kurzer Zeit ein Maximum erreicht. Insbesondere bevorzugt ist die Kombiniation des erfindungsgemaßen Weißdornbeerenextraktes mit Kampfer.

Die folgenden Beispiele sollen die Erfindung näher erläutern, ohne sie zu beschränken.

### Beispiel 1: Herstellung eines Weißdornbeerenextraktes

100 g frische, reife Weißdornbeeren (Crataegus monogyna) werden geerntet und im frischen oder tiefgefrorenen Zustand mit 100 g 96 %igem Ethanol versetzt, zu einem Brei zerschlagen und durch herkömmliche Mazeration oder Bewegungsmazeration ausgezogen. Nach Filtration und Abpressen erhält man einen Fluidextrakt mit einem Droge-Extrakt-Verhältnis von etwa 1 : 1,4.

Dann wird das Lösungsmittelgemisch des Fluidextraktes unter vermindertem Druck verdampft, bis der verbleibende Extrakt ein Droge-Extrakt-Verhältnis von 5 : 1 aufweist. Der Gehalt an oligomeren Procyanidinen, als Cyanidin-Hydrochlorid berechnet, beträgt etwa 3 %.

### Beispiel 2: Wirkungsstudie

In einer placebokontrollierten Studie wurden die kardiovaskulären Wirkungen des in Beispiel 1 hergestellten Weißdornbeerenextraktes untersucht.

### Prinzip:

14 gesunde, männliche Probanden im Alter von 18 - 45 Jahren nahmen über einen Zeitraum von 4 Wochen an den Studientagen 1 und 28 1 x 360 Tropfen des Weißdornbeerenextraktes, was 45 g frischen Weißdornbeeren ( 270 mg Procyanidin-Äquivalenten) entsprach, und an den Studientagen 2 - 27 3 x 120 Tropfen des Weißdornbeerenextraktes, was 3 x 15 g frischen Weißdornbeeren (3 x 90 mg Procyanidin-Äquivalenten) entsprach, ein. Für die Behandlung mit Placebo wurde der Weißdornbeerenextrakt durch eine 40 Vol-%ige Ethanollösung ersetzt.

An den Hauptstudientagen 1 und 28 wurden zu den Zeitpunkten 0:05; 0:15; 0:30; 1:00; 1:30; 2:00; 2:30; 3:00; 4:00; 5:00 und 6:00 h nach Einnahme des Weißdornbeerenextraktes die nachfolgend beschriebenen Untersuchungen vorgenommen.

### Experimentelles:

Es wurde eine 4-Kanal-Kreislauf- und Blutdruckanalyse vorgenommen, welche die simultane Registrierung von EKG, Herzschall, Carotispulskurve und transthorakaler, differenzierter Impedanzkardiographie (Cardio-Dynagraph® Diefenbach) umfaßte. Die Messungen wurden in expiratorischer Atemruhe durchgeführt. Zu jedem Meßzeitpunkt erfolgte die Registrierung von 25 Komplexen bei einer Schreibgeschwindigkeit von 10 mm/s unmittelbar gefolgt von 10 Komplexen bei einer Schreibgeschwindigkeit von 100 mm/s (4-Kanal-Schreiber Cardirex 3T Siemens Elema). Zu jedem Zeitpunkt der 4-Kanal-Kreislaufanalyse wurde auch der Blutdruck auskultatorisch bestimmt (Korotkov I und V). Durch die Kombination mit einem Hestia OZ80® Blutdruckmeßgerät konnte die Herzfrequenz gleichzeitig abgelesen werden.

Die Auswertung der gewonnenen Daten erfolgte einerseits manuell (halbdigitale Auswertung), andererseits mit Hilfe eines analogen Digital-Wandlers. Zu diesem Zweck wurden die entsprechenden Zeitintervalle und Signalamplituden auf den analogen 4-Kanal-Registrierungen markiert und der automatischen Datenverarbeitung zugeführt. Die entsprechenden Blutdruck- und transthorakalen Grundimpedanzwerte wurden manuell eingegeben, die weitere Datenverarbeitung verlief automatisch.

### Resultate:

Beim systolischen Blutdruck zeigten sich schon am ersten Tag der Anwendung tendenzielle Steigerungen, die sich nach vierwöchiger Einnahme signifikant nachweisen ließen. Nach 28 Tagen lagen die Basiswerte ohne morgendlich eingenommene Medikation bereits höher als die vergleichbare Basislinie des Tages 1, was unter Placebogabe nicht beobachtet werden konnte. Im Vergleich zur Placebobehandlung lagen die systolischen Blutdruckwerte in den ersten zwei Stunden nach Einnahme des Weißdornbeerenextraktes um ca. 10 mmHg höher. Die Hauptwirkung war bis zu 3 Stunden nach Einnahme feststellbar. Selbst nach 6 Stunden war das durchschnittliche systolische Blutdruckniveau noch leicht erhöht.

Auch der diastolische Blutdruck war am Tag 28 signifikant erhöht. Ähnlich wie beim systolischen Blutdruck lag auch hier das Niveau nach 6 Stunden immer noch signifikant höher als unter Placeboeinnahme. Dies ließ auf eine längerfristig andauernde Wirkung des Weißdornbeerenextraktes schließen. Die Basiswerte ohne erneute morgendliche Einnahme lagen nach 4 Wochen höher als die ursprünglichen Ausgangswerte ohne Einnahme am Tag 1.

Analog zu den oben beschriebenen Blutdruckeffekten ließen sich auch für den arteriellen Mitteldruck signifikante Drucksteigerungen nach Einnahme des Weißdornbeerenextraktes nachweisen. Die Hauptwirkung war innerhalb der ersten 2 bis 3 Stunden feststellbar. Am Tag 28 lagen die Basiswerte schon vor Einnahme deutlich höher als unter Placebo, und das Kurvenniveau blieb auch noch nach 6 Stunden leicht erhöht.

Beim totalen peripheren Widerstand war ein statistisch signifikanter Anstieg sowohl am Tag 1 als auch nach 4-wöchiger Einnahme des Weißdornbeerenextraktes nachweisbar.

Bezüglich der Herzfrequenz war weder am Tag 1 noch am Tag 28 der Behandlung ein signifikanter Unterschied nach Einnahme des Weißdornbeerenextraktes bzw. des Placebos feststellbar.

Auch beim Schlagvolumen, dem Cardiac-Output und dem Heather-Index waren keine signifikanten Veränderungen nachweisbar.

Die thorakale Grundimpedanz dagegen lag nach 4-wöchiger Einnahme des Weißdornbeeren-Extraktes deutlich höher als unter Placeboeinnahme.

Keine eindeutig gerichteten Veränderungen waren für die elektromechanische Systolendauer (frequenzkorrigiert) und die Präejektionsperiode feststellbar. Eine Verkürzung der linksventrikulären Ejektionszeit konnte an Versuchstag 28 festgestellt werden, die nicht nur während der ersten zwei Stunden ausgeprägt war sondern bis zum Ende des Versuchstages signifikant erhalten blieb.

### Schlußfolgerung:

In Anbetracht der obigen Resultate ließ sich feststellen, daß die Einnahme des Weißdornbeerenextraktes sowohl in der akuten als auch in der chronischen Anwendung deutliche Veränderungen bei den verschiedenen Herz-Kreislauf-spezifischen Parametern zur Folge hatte. Die beobachteten Blutdruckerhöhungen waren wahrscheinlich auf einen erhöhten totalen peripheren Widerstand zurückzuführen. Aufgrund der Verkürzung der linksventrikulären Ejektionszeit konnte auf eine Leistungssteigerung des Herzens geschlossen werden. Der Anstieg der thorakalen Grundimpedanz wies auf eine höhere intrakorporale Electrolytdichte bzw. einen geringeren Flüssigkeitsgehalt hin und stand somit für eine diuretische Wirkung des untersuchten Weißdornbeerenextraktes.

## Patentansprüche

1. Verwendung eines Weißdornfrüchte- und/oder -blätter/blütenextraktes zur Steigerung der Leistungsfähigkeit des Herzens und gleichzeitigen Erhöhung des Blutdrucks.

2. Verwendung nach Anspruch 1 zur Behandlung von Herzbeschwerden bei Hypotonie.

3. Verwendung eines Weißdornfrüchte- und/oder -blätter/blütenextraktes zur Förderung der Diurese.

4. Verwendung nach Anspruch 3 zur Förderung der Diurese bei Hypotonie.

5. Verwendung nach Anspruch 3 oder 4 zur Behandlung von Ödemen und/oder hydropischen Zuständen unterschiedlicher Genese.

6. Verwendung nach einem der vorhergehenden Ansprüche, wobei der Extrakt eine Tagesdosis von 15 bis 1200 Procyanidin-Äquivalenten, vorzugsweise von 36 bis 600 Procyanidin-Äquivalenten, umfaßt.

7. Verwendung nach einem der vorhergehenden Ansprüche, in oral verabreichbarer Form, vorzugsweise als Tropfen oder Tabletten.
